Europäisches Patentamt

European Patent Office

Office européen des brevets

(11) Veröffentlichungsnummer: **0 554 435 B1**

(12) **EUROPÄISCHE PATENTSCHRIFT**

(45) Veröffentlichungstag der Patentschrift: **20.12.95**

(51) Int. Cl.6: **A23L 1/305**

(21) Anmeldenummer: **92918419.0**

(22) Anmeldetag: **26.08.92**

(86) Internationale Anmeldenummer:
**PCT/EP92/01966**

(87) Internationale Veröffentlichungsnummer:
**WO 93/03633 (04.03.93 93/06)**

(54) **VERFAHREN ZUR HERSTELLUNG EINES PHENYLALANINFREIEN DIÄTETIKUMS IN DRAGEE- ODER TABLETTENFORM**

(30) Priorität: **26.08.91 DE 4128260**

(43) Veröffentlichungstag der Anmeldung:
**11.08.93 Patentblatt 93/32**

(45) Bekanntmachung des Hinweises auf die
Patenterteilung:
**20.12.95 Patentblatt 95/51**

(84) Benannte Vertragsstaaten:
**AT BE CH DE DK ES FR GB GR IE IT LI LU NL**

(56) Entgegenhaltungen:
**EP-A- 0 198 431**
**EP-A- 0 488 078**
**DE-C- 863 986**
**FR-A- 2 574 254**
**US-A- 3 764 703**

**CHEMICAL ABSTRACTS, vol. 98, no. 17, April 1983, Columbus, Ohio, US; abstract no. 142181g, 'protein hydrolysate free of salt and phenylalanine' Seite 474 ;Spalte 2 ;**

(73) Patentinhaber: **MILUPA AKTIENGESELL-SCHAFT**
**Bahnstrasse 14-30**
**D-61381 Friedrichsdorf (DE)**

(72) Erfinder: **WACHTEL, Ursula**
**Promenade 140 a**
**D-6380 Bad Homburg v.d.H. (DE)**
Erfinder: **SCHWEIKHARDT, Friedrich**
**Falkenweg 1**
**D-6382 Friedrichsdorf/Ts.-2 (DE)**
Erfinder: **TESMER, Erhard**
**Bergstrasse 2a**
**D-7600 Offenburg/Baden (DE)**

(74) Vertreter: **Köster, Hajo, Dr. et al**
**Jaeger, Böck & Köster,**
**Patentanwälte,**
**Postfach 16 20**
**D-82131 Gauting b. München (DE)**

**Beschreibung**

Die Erfindung betrifft ein phenylalaninfreies Diätetikum auf Basis von Aminosäuren für Personen, insbesondere juvenile und adulte Personen sowie schwangere Frauen, die unter einer Phenylketonurie leiden in Dragee- oder Tabletten form, ein Verfahren zur Herstellung dieses Phenylalaninfreien Diätetikums sowie eine dabei einsetzbare Masse.

Die Phenylketonurie (nachstehend PKU genannt) gehört zu den genetisch bedingten Erkrankungen, und stellt eine Stoffwechselstörung dar.

Als Folge der Stoffwechselstörung häuft sich bei den erkrankten Personen bzw. Patienten das Phenylalanin im Körper an, so daß sich dessen Gehalt im Blut und im Gewebe weit über den Normalbereich hinaus erhöht.

Der erhöhte Phenylalaninspiegel im Plasma führt zu einer Beeinträchtigung zahlreicher Stoffwechselvorgänge im Gehirn. Wird die PKU nicht behandelt, kommt es bei Kindern zu einer Hirnreifestörung mit geistigem Entwicklungsrückstand in unterschiedlicher Ausprägung.

Die Phenylketonurie kann mit einer phenylalaninarmen Ernährungsweise mehr oder weniger erfolgreich behandelt werden. Die Patienten entwickeln sich daher physisch und psychisch normal und sind bildungsfähig. Zudem erreichen sie problemlos das Fortpflanzungsalter, so daß heute zunehmend junge PKU-Patientinnen schwanger werden.

Um bei einem unter der PKU leidenden Kind eine normale geistige und körperliche Entwicklung zu erzielen, muß sein Phenylalaninspiegel im Plasma mit einer phenylalaninarmen Diät auf Normalwerte gesenkt und stabilisiert werden.

Dazu bekommen derartige Kinder eine Nahrung, die eine beschränkte Menge an natürlichem Eiweiß und gerade soviel Phenylalanin enthält, wie der kindliche Körper zum Aufbau von Eiweiß (Wachstum) benötigt. Für die Ernährung können daher nur solche Lebensmittel eingesetzt werden, die von Natur aus einen niedrigen Eiweißgehalt und damit auch einen niedrigen Phenylalaningehalt besitzen.

Mit einer derartigen phenylalaninarmen Ernährung allein würde den Kindern jedoch zu wenig von allen anderen, für das Leben ebenso wichtigen Aminosäuren zugeführt werden. Daher benötigen Kinder, die an der PKU leiden, zusätzlich zu einer phenylalaninarmen Diät eine Eiweißquelle, die zwar kein Phenylalanin, jedoch aber eine ausreichende Menge aller anderen Aminosäuren enthält.

Es sind bereits Spezialerzeugnisse bekannt, die aus Eiweißbausteinen, den Aminosäuren, zusammengesetzt sind, jedoch kein Phenylalanin enthalten. Diesen Mischungen sind zusätzlich Vitamine, Mineralstoffe und Spurenelemente einverleibt, da ein unter der PKU leidendes Kind diese Nährstoffe mit der phenylalaninarmen Ernährung allein nicht in ausreichenden Mengen erhalten würde.

Da heutzutage immer mehr empfohlen wird, daß unter der PKU leidende Patienten die phenylalaninarme Diät lebenslang beibehalten und da eine derartige Diät insbesondere schwangeren, an der PKU leidenden Frauen empfohlen wird, müssen auch diese Patientengruppen die PKU-Diät einhalten sowie die bekannten Spezialprodukte zu sich nehmen.

Bisher wurde nun die Auffassung vertreten, daß der Eiweißstoffwechsel die gleichzeitige Anwesenheit aller und somit auch der nicht essentiellen Aminosäuren erfordert. Aus diesem Grunde enthalten die bekannten und üblicherweise eingesetzten Spezialprodukte bzw. phenylalaninfreien Aminosäuremischungen für Säuglinge und Kinder mit ihrem hohen Wachstumsbedarf auch alle Aminosäuren mit Ausnahme des in seinem Stoffwechsel gestörten Phenylalanins.

In Abhängigkeit von der individuellen Stoffwechselsituation eines unter der PKU leidenden Patienten mußte dieser pro Tag 45 bis 70 g der bekannten phenylalaninfreien Aminosäurenmischung zu sich nehmen. Dies stellt eine erhebliche Stoffmenge dar, welche die Akzeptanz dieser bekannten Spezialprodukte durch die Patienten, insbesondere durch juvenile Patienten, stark negativ beeinflusst.

In der nicht vorveröffentlichten deutschen Patentanmeldung P 40 37 447.5 ist ein phenylalaninfreies Diätetikum beschrieben, das nur bestimmte Aminosäuren enthält. Der tägliche Eiweißbedarf von jugendlichen und erwachsenen, unter der PKU leidenden Patienten, kann nämlich auch mit einer Aminosäurenmischung vollständig oder fast vollständig gedeckt werden, die nur bestimmte Aminosäuren enthalten. Dadurch wird die täglich einzunehmende Menge reduziert. Dieses in der genannten deutschen Patentanmeldung beschriebene Diätetikum stellt im übrigen ein Pulver dar.

Die Akzeptanz einer Diät - dies gilt besonders für adoleszente Personen - und eines zur Unterstützung dieser Diät einzunehmenden Diätetikums hängt zum großen Teil davon ab, wie stark und wie schnell dies gegenüber der Umwelt im außerhäuslichen Bereich manifest wird.

An der PKU leidende Personen müssen mit der Mahlzeit eine beträchtliche Menge einer phenylalaninfreien Aminosäurenmischung zu sich nehmen. Die bisher bekannten Aminosäurenmischungen und entsprechenden Spezialprodukte besitzen einen fremdartigen und unangenehmen Geruch, der von umgebenden Personen als abstoßend empfunden wird. Außerdem besitzen diese Aminosäurenmischungen einen spezifischen sowie störenden Geschmack.

Aus letzteren Gründen brechen daher eine große Zahl der PKU-Patienten die genannte Diät ab und nehmen auch die phenylalaninfreien Aminosäurenmischungen nicht mehr zu sich.

Aus der US-A-3764 703 sind bereits aminosäurehaltige Tabletten mit einem den unangenehmen Geschmack der Aminosäuren abdeckenden Überzug bekannt.

Aufgabe der vorliegenden Erfindung ist es, ein Verfahren zur Herstellung eines phenylalaninfreies Diätetikum sowie ein derartiges Diätetikum bereitzustellen, das von unter der PKU leidenden Patienten besser akzeptiert wird.

Gelöst wird diese Aufgabe hinsichtlich des Verfahrens durch die Lehre des Anspruchs 1 sowie hinsichtlich des Diätetikums durch die Lehre des Anspruchs 9.

Erfindungsgemäß wird ein phenylalaninfreies Diätetikum bzw. eine phenylalaninfreie Aminosäurenmischung bereitgestellt, die nach dem erfindungsgemäßen Verfahren erhältlich ist, die alle oder einen Teil der für die Ernährung erforderlichen Aminosäuren oder nur die essentiellen Aminosäuren enthält und die frei von dem charakteristischen Eigengeschmack und -geruch von den bisher bekannten Aminosäurenmischungen ist. Das erfindungsgemäße Diätetikum kann außerdem noch Vitamine, Mineralstoffe und/oder Spurenelemente enthalten, um die Versorgung mit diesen essentiellen Nährstoffen sicherzustellen.

Das erfindungsgemäße phenylalaninfreie Diätetikum liegt in Form von Dragees oder Tabletten vor. Die Dragees bzw. Tabletten sind dabei mit einem Überzug bzw. einer Dragierdecke versehen oder überzogen, so daß der Geschmack und der Geruch der im Tabletten- bzw. Drageekern vorhandenen L-Aminosäuren von den Patienten nicht mehr bemerkt wird.

Da die Dragees häufig lebensbegleitend Anwendung finden, muß die Überzugsmasse frei von Stoffen mit pharmakologischer Wirkung sein. Daher werden für Arzneimittel zugelassene Stoffe nicht eingesetzt, Vielmehr besteht die Überzugsmasse nur aus solchen Stoffen, die lebensmittelrechtlich unbedenklich sind. Auch sollten diese Stoffe derart gewählt sein, daß die Dragees wegen der besseren Verträglichkeit möglichst erst im Dünndarm vollständig zerfallen.

Zweckmäßigerweise wählt man eine Überzugsmasse, die einen neutralen oder angenehmen Eigengeschmack besitzt.

Die Dragee- bzw. Tablettenkerne (nachstehend steht der Ausdruck "Dragees" auch für Tabletten) sind zweckmäßigerweise mit einer Dragierdecke aus Zucker und Zusatzstoffen überzogen, die als solche zur Herstellung von Lebensmitteln zugelassen sind. In Deutschland sollten somit nur Stoffe Anwendung finden, die gemäß dem Lebensmittelgesetz und dem Bedarfsgegenständegesetz zugelassen sind. Diese Dragierdecke deckt den Geruch und den Geschmack der in den Drageekernen vorhandenen Aminosäuren bzw. Wirkstoffmischung so ab, daß die Dragees geschmacksneutral sind.

Das erfindungsgemäße Diätetikum in Drageeform stellt für die betroffen, an der PKU leidenden Patienten eine außerordentliche Erleichterung bei der praktischen Durchführung einer langdauernden bzw. lebenslänglich durchzuführenden Diät dar.

Die Dragees können leicht und problemlos über den ganzen Tag verteilt, in der vom Arzt verordneten Menge mit Wasser, Tee, Saft oder ähnlichen Getränken eingenommen werden. Es erübrigt sich eine Zubereitung durch Auflösen von Pulvern und Granulaten in Flüssigkeiten, wie das bei den bisher bekannten Spezialprodukten erforderlich war. Dadurch ist die Anwendung einfach, die Dosierung sicher und die Einstellung des Plasmaphenylalaninspiegels im Blut leicht möglich.

Bei schwangeren Patientinnen kann durch das erfindungsgemäße Diätetikum vermieden werden, daß durch vermehrtes Erbrechen die Energie- und Nährstoffversorgung beeinträchtigt wird. Die geschmacksneutralen Dragees verursachen keinen Brechreiz wie die bisher bekannten pulverförmigen Aminosäurenmischungen; sie werden gut vertragen und ermöglichen so während der Gravidität eine sichere Durchführung der PKU-Diät.

Damit die L-Aminosäuren, die ggf. zusammen mit Kohlenhydraten, Mineralstoffen, Spurenelementen und/oder Vitaminen zusammen vorliegen bzw. damit vermischt sind, zu Dragees verarbeitet werden können, ist es wesentlich, daß dieser Masse, die gewöhnlicherweise als Pulver vorliegt, eine als Trennmittel dienender Fettstoff beigemengt ist. Gegenstand der Erfindung ist somit auch eine derartige Masse bzw. ein derartiges Halbfabrikat.

Bedingt dadurch, daß das in Drageeform vorliegende Diätetikum in dem Kern des Dragees einen derartigen Fettstoff aufweist, ist es möglich, diesen Kern mit einem Überzug bzw. einer Drageedecke zu versehen.

Beim erfindungsgemäßen Verfahren werden in Stufe a) die L-Aminosäuren und die ggf. vorhandenen Mineralstoffe und/oder Spurenelemente in Wasser gegeben und dabei gelöst bzw. dispergiert. Der so erhaltene Naßansatz wird dann anschließend sprühgetrocknet. Das Sprühprodukt wird dann in Stufe d) zu Dragee- oder Tablettenkernen verarbeitet, die dann anschließend in Stufe e) mit einem Überzug oder einer Drageedecke versehen werden.

Es hat sich nun gezeigt, daß ein derartiges Sprühprodukt ohne Trennmittel nur schwer kontinuierlich verarbeitet werden kann, da es sonst zu Anbackungen in der Tablettenpresse kommt. Daher setzt man einen Fettstoff zu, der als Trennmittel dient.

Die Gesamtmenge dieses Fettstoffes kann man der zu Drageekernen zu verarbeitenden Masse einverleiben, indem man diesen Fettstoff mit dem in Stufe b) erhaltenen Sprühprodukt vermischt.

Als Trennmittel bzw. als Fettstoff haben sich hochschmelzende Mono- und/oder Diglyceride und Magnesiumstearat bewährt. Es ist jedoch auch möglich, andere, Trennmittelfunktionen habende Fettstoffe zur Anwendung zu bringen.

Nach einer weiteren Ausführungsform ist es möglich, die Gesamtmenge des Fettstoffes bereits in Stufe a) der sprühzutrocknenden wässrigen Lösung bzw. Dispersion einzuverleiben. In diesem Fall fungiert der Fettstoff nicht nur beim Tablettieren als Trennmittel, sondern verbessert auch die Verteilung der Inhaltsstoffe im Naßansatz vor der Sprühtrocknung und hebt die Benetzbarkeit an. Vorzugsweise werden deshalb Emulgatoren mit hohem Schmelzpunkt (70°C), hochschmelzende Fette oder ähnliche Fettstoffe eingesetzt, die lebensmittelrechtlich zur Herstellung von Lebensmitteln, insbesondere diätetischen Lebensmitteln zugelassen sind.

Vorzugsweise wird jedoch eine Teilmenge des Fettstoffes dem sprühzutrocknenden Naßansatz hinzugegeben, während eine weitere Teilmenge des Fettstoffes mit dem Sprühprodukt vermischt wird. Bei letzterer Ausführungsform ist es möglich, dem Naßansatz einen Feststoff einzuverleiben, der sich von dem mit dem Sprühprodukt zu vermischenden Fettstoff unterscheidet. Bei allen beschriebenen Ausführungsvarianten ist es natürlich möglich, eine Fettstoffmischung zur Anwendung zu bringen.

Auch die ggf. vorhandenen Vitamine und/oder die ggf. vorhandenen Kohlenhydrate können insgesamt dem Naßansatz zugegeben werden oder mit dem Sprühprodukt vermischt werden. Auch in diesem Fall ist es möglich, eine Teilmenge der Vitamine und/oder Kohlenhydrate dem Naßansatz und die restliche Teilmenge dem Sprühprodukt beizugeben. Vorzugsweise gibt man die gesamte Menge der Vitamine erst nach der Sprühtrocknung hinzu bzw. vermischt sie mit dem Sprühprodukt. Die Vitamine sind dabei vorzugsweise mit einem oder mehreren Kohlenhydraten vorvermischt. Weitere Kohlenhydrate können in letzterem Fall bereits dem Naßansatz zugegeben worden sein.

Die Kohlenhydrate können verschiedener Herkunft sein. Vorzugsweise setzt man aus technologischer Sicht jedoch Zucker, Milchzucker, Dextrose, Maltodextrine und Stärke ein. Die den Tablettenkernen beigemengten Kohlenhydrate stellen Hilfsmittel zur Tablettierung dar. Außerdem stellen sie natürlich Drageedeckensubstanzen dar.

Die so gewonnene Pulvermischung bzw. Masse kann man direkt zu Tabletten verarbeiten bzw. verpressen Vorzugsweise kompaktiert man jedoch vor, granuliert und verpreßt dann zu Tabletten- bzw. Drageekernen, die anschließend mit einem Überzug oder einer Drageedecke versehen werden.

Um eine reibungslose Produktion zu gewährleisten, presst man die so erhaltene Pulvermischung vorzugsweise zunächst in einem Kompaktor und granuliert anschließend vorzugsweise mit einem Frewitt-Granulator oder einem anderen geeigneten Brechersystem.

Weiterhin bevorzugt führt man die Verarbeitung der Pulvermischung bzw. der zu Tabletten zu verarbeitenden Masse in einem Raum durch, in dem die relative Luftfeuchtigkeit ≦ 30 % und insbesondere 20 % oder weniger ist.

Bei der Herstellung des Sprühproduktes läßt man den pH-Wert des Naßansatzes vorzugsweise nicht unter 6,3 absinken. Vorzugsweise kontrolliert man daher während der Produktion laufend den pH-Wert und gibt ggf. alkalisch machende Verbindungen, vorzugsweise eine Kaliumhydroxid- oder Kaliumcarbonatlösung zu dem Naßansatz, um den pH-Wert bei mindestens 6,3 zu halten.

Die in dem erfindungsgemäßen Diätetikum vorhandenen Aminsoäuren bzw. die beim erfindungsgemäßen Verfahren eingesetzten Aminosäuren können in jeder geeigneten Form, insbesondere in jeder für Lebensmittelzwecke zulässigen Form vorliegen. Die Aminosäuren können beispielsweise als Salze, Hydrochloride, Hydrate, Acetate und Malate etc. vorliegen. Auch können die Aminosäuren in Form von Dipeptiden eingesetzt werden, sofern diese Dipeptide kein Phenylalanin enthalten.

Da L-Glutaminsäure und L-Asparaginsäure sehr sauer reagierende Aminosäuren sind, werden diese vorzugsweise in Form der Na-, K- oder Mg-Salze oder als L-Lysin-L-Glutamat oder L-Arginin-L-Glutamat eingesetzt, da sonst ein dramatischer Abfall des pH-Wertes ds Naßansatzes bis unter 5,0 erfolgen kann. Dies kann zu erheblichen Instabilitäten und Phasentrennungen während der Bereitung des Naßansatzes führen. Die Aminosäuren werden im übrigen vorzugsweise in einer bestimmten Reihenfolge zum Naßansatz hinzugegeben, da dadurch Fällungen und Inhomogenitäten durch Sedimentation bei der Herstellung des Sprühproduktes vermieden werden können. So gibt man vorzugsweise die nachfolgend aufgeführten

4

Aminosäuren in der folgenden Reihenfolge zu: Natrium-L-Glutamat, L-Arginin-L-Glutamat, L-Lysin-L-Glutamat, K-L-Aspartat, Mg-L-Aspartat, L-Prolin, L-Isoleucin, L-Leucin, L-Tyrosin, L-Threonin, L-Valin, L-Alanin, L-Methionin, L-Cystin, L-Histidin, L-Glycin, L-Tryptophan, L-Serin.

Nach einer weiterhin bevorzugten Ausführungsform setzt man zur Herstellung des Naßansatzes kaltes Wasser, insbesondere von 3 bis 15°C ein und löst in diesem kalten Wasser zunächst ein die Viskosität anhebendes Mittel, beispielsweise Guar, um dadurch zu vermeiden, daß wasserunlösliche Stoffe sedimentieren. Danach arbeitet man die Aminosäuren, die ggf. vorhandenen Mineralstoffe und/oder Spurenelemente und den gewünschten Anteil der Kohlenhydrate ein. Anschließend erhitzt man den Naßansatz auf insbesondere 70 bis 90°C, vorzugsweise 75 - 80°C und am meisten bevorzugt ca. 75°C. Anschließend gibt man erst die gewünschte Menge des Fettstoffes hinzu. Der Grund für diese Vorgehensweise ist folgender: Bekanntlich beginnen Zucker und Aminosäuren bereits bei Temperaturen über 70°C Bräunungsprodukte zu bilden (Maillard-Reaktionen). Um diese Maillard-Reaktionen so niedrig wie möglich zu halten, wird der Ansatz - wie oben geschildert - kalt bereitet und erst vor Zugabe der Fettstoffe bzw. Emulgatoren erwärmt. Demzufolge ist es von Bedeutung, Fettstoffe bzw. Emulgatoren erst zum Schluß in den heißen Ansatz einzubringen.

Das erfindungsgemäße phenylalaninfreie Diätetikum ist somit vorzugsweise dadurch erhältlich, daß man alle L-Aminosäuren, die ggf. vorhandenen Mineralstoffe und/oder Spurenelemente, einen Teil der Fettstoffe bzw. Emulgatoren und der Kohlenhydrate in Wasser gibt und dabei dispergiert, löst bzw. emulgiert. Dabei hält man vorzugsweise die oben beschriebenen Verfahrensparameter hinsichtlich pH-Wert, Temperatur und Reihenfolge ein. Im Anschluß daran homogenisiert man, um ein möglichst gleichmäßiges, glattes, viskoses und homogenes Konzentrat zu haben. Anschließend pasteurisiert man, zweckmäßigerweise mit einem Schabeerhitzer, wodurch eine starke Keimreduktion des Konzentrates erfolgt. Danach wird das Produkt nachhomogenisiert und auf einem Sprühturm, vorzugsweise mit Düsen, getrocknet. Das Sprühprodukt vermischt man mit der Vitaminmischung (vorzugsweise mit der Gesamtmenge), die vorzugsweise in einer Teilmenge der insgesamt eingesetzten Kohlenhydrate vorgemischt ist. Diese Pulvermischung vermischt man dann vorzugsweise mit einer Teilmenge der insgesamt vorhandenen Fettstoffe.

Es hat sich gezeigt, daß die Rückführung von Zyklonpulver und eines Teils des Sprühpulvers in dem Sprühturm möglichst niedrig zu halten ist, um ein Ankleben und eine zu starke Instantisierung zu vermeiden. Das so gewonnene Pulver hat gute Fließeigenschaften.

Das zur Herstellung der Dragee- bzw. Tablettenkerne dienende Pulver bzw. Granulat läßt sich ohne Anbackungen zu verursachen in Tablettenpressen (Rundläufer, Exzenterpressen und ähnlichem) zu Tablettenkernen verarbeiten.

Diese Kerne dragiert man dann mit Zucker, Geliermittel wie Pektin und Gummi arabicum. Vorzugsweise isoliert man die hygroskopischen Tablettenkerne vor Aufbringen der Drageedecke sorgfältig, da es sonst im Verlauf der Lagerung zu Rissen in der Drageedecke kommen kann.

Zur Isolierung der Kerne verwendet man vorzugsweise fettartige Substanzen, wie z.B. Boeson® VP, und Geliermittel. Diese Substanzen fungieren auch als Klebemittel für die Drageedecke. Vorzugsweise werden bei der Herstellung der Drageedecke nur Zusatzstoffe verwendet, die zur Herstellung von Lebensmitteln gemäß Lebensmittel- und Bedarfsgegenständegesetz zulässig sind.

Es hat sich gezeigt, daß mit Zucker und Geliermittel wie Pektin und Gummi arabicum eine stabile, reißfeste Drageedecke erhalten werden kann. Diese Drageedecke ist geschmacksneutral bzw. beim Anlutschen süß und deckt den Eigengeruch der Wirkstoffe vollständig ab.

Vorzugsweise stellt man Dragees mit einem Gewicht von 700 bis 800 mg her, wobei der Wirkstoffgehalt (Aminosäuren plus weitere Bestandteile der Kerne) 400 mg ausmacht.

Die Menge an einzunehmenden Dragees richtet sich nach der Wirkstoffmenge, die pro Tag eingenommen werden muß; diese kann bei 15 bis 40 Dragees/Tag liegen.

Das erfindungsgemäß bzw. erfindungsgemäß erhältliche phenylalaninfreie Diätetikum ist ein Suplement zur Ernährung, das nach ärztlicher Verordnung dosiert wird. Es ist deshalb nicht erforderlich, das Produkt mit Fett anzureichern.

Die Erfindung wird im folgenden anhand in den Beispielen näher beschriebenen bevorzugten Ausführungsformen weiter erläutert.

Beispiel 1

Herstellung eines phenylalaninfreien Diätetikums in Drageeform

|  | A Gew.-% | B Gew.-% |
|---|---|---|
| L-Histidin | 1,708 | 1,716 |
| L-Isoleucin | 4,270 | 4,291 |
| L-Leucin | 7,212 | 7,247 |
| L-Lysin-L-Glutamat | 11,673 | 11,729 |
| L-Methionin | 1,708 | 1,716 |
| L-Threonin | 3,417 | 3,433 |
| L-Tryptophan | 1,329 | 1,335 |
| L-Valin | 5,124 | 5,149 |
| L-Alanin | 2,942 | 2,956 |
| L-Arginin-L-Glutamat | 4,840 | 4,863 |
| Magnesium-L-Aspartat | 1,993 | 2,003 |
| L-Cystin | 1,708 | 1,716 |
| Na-L-Glutamat | 9,130 | 9,174 |
| L-Glycin | 1,708 | 1,716 |
| L-Prolin | 6,738 | 6,770 |
| L-Serin | 3,796 | 3,814 |
| L-Tyrosin | 5,694 | 5,721 |
| Kalium-L-Aspartat | 7,160 | 7,194 |
| Vanillin | 0,076 | 0,076 |
| Guar | 0,949 | 0,954 |
| Monoglyzerid (Emulgator) | 0,949 | 0,954 |
| Kaliumchlorid | 0,995 | 1,000 |
| Kaliumhydrogenphosphat | 1,624 | 1,632 |
| Magnesiumhydrogenphosphat | 1,692 | 1,700 |
| Natriumhydrogenphosphat | 1,054 | 1,059 |
| Eisensulfat(II) | 0,099 | 0,099 |
| Kupfer-II-Acetat | 0,011 | 0,011 |
| Zinksulfat | 0,062 | 0,062 |
| Mangansulfat | 0,014 | 0,014 |
| Kaliumjodid | 0,00036 | 0,00036 |
| Ammoniumheptamolybdat | 0,00084 | 0,00084 |
| Magnesiumcitrat | 1,40300 | 1,41000 |
| Myo-Inosit | 0,28500 | 0,28600 |
| Cholinhydrogentartrat | 0,51700 | 0,51700 |
| Vitaminmischung | 0,47700 | --- |
| Zucker | 7,64180 | 7,67980 |
|  | 100,00000 | 100,00000 |

Tabelle A = Rezeptur

Tabelle B = Rezeptur für das Sprühprodukt

Aus diesen Komponenten mit Ausnahme der Vitaminmischung wird zunächst ein sprühgetrocknetes Zwischenprodukt hergestellt. Die Zusammensetzung (in Gew.-%) des Sprühproduktes ist in Spalte B wiedergegeben.

1.1 Zur Herstellung einer 100-kg-Charge werden 97 l kaltes Wasser in einen heizbaren Mischtank mit Intensivrührwerk (Y-Stral, Ultraturrax) gegeben.

In diesem kalten Wasser werden zunächst 954 g Guar vollständig gelöst. Danach werden 1 kg Kaliumchlorid in ca. 3 l warmem Wasser vorgelöst und der Guarlösung zugesetzt.

Kaliumhydrogenphosphat und Natriumdihydrogenphosphat werden separat in jeweils ca. 6,5 l Wasser vorgelöst und nacheinander dem Ansatz zugesetzt. Danach wird Myo-Inosit in ca. 3 l Wasser vorgelöst und dem Ansatz zugegeben. Der Ansatz wird intensiv gerührt und im Kreislauf gepumpt.

Natrium-L-Glutamat wird danach unter Verwendung einer Venturi-Düse in den Ansatz eingearbeitet. Der pH-Wert der Lösung wird kontrolliert und sollte zwischen 6.6 - 7.0 liegen.

Danach werden unter Verwendung einer Venturi-Düse nacheinander die nachstehend genannten Rohstoffe unter intensivem Rühren eingearbeitet.

L-Arginin-L-Glutamat

L-Lysin-L-Glutamat

Kalium-L-Aspartat

Magnesium-L-Aspartat

L-Prolin

L-Isoleucin

L-Leucin

L-Tyrosin

L-Valin

L-Threonin

L-Alanin

L-Methionin

L-Cystin

L-Histidin

L-Glycin

L-Tryptophan

L-Serin

Magnesiumhydrogenphosphat

Magnesiumcitrat

Das Eisensulfat wird in 2 l Wasser vorgelöst und dem Ansatz zugesetzt. Kupferacetat wird in ca. 300 ml Wasser aufgelöst und dem Ansatz zugegeben. Zinksulfat wird in ca. 650 ml Wasser vorgelöst und dem Ansatz zugesetzt.

Mangansulfat wird in 650 ml Wasser vorgelöst und dem Ansatz zugegeben. Kaliumjodid und Ammoniumheptamolybdat werden separat in jeweils in ca. 60 ml heißem Wasser (60°C) vorgelöst und nacheinander dem Ansatz zugesetzt.

Der gesamte Ansatz wird unter intensivem Rühren auf 75°C erwärmt, wobei gleichzeitig der Ansatz kontinuierlich im Kreislauf gepumpt wird.

Der pH-Wert des Ansatzes bewegt sich zwischen 6.3 - 6.5 Falls der pH-Wert unter 6.3 absinkt, wird eine 1-n-Kalilauge oder eine 10%ige wässrige Kaliumkarbonatlösung hinzugegeben, bis der pH wieder gleich oder größer als 6.3 ist. In einem separaten Gefäß wird der Emulgator im Wasserbad aufgeschmolzen und dem Ansatz zugesetzt, der eine Temperatur von 75°C haben sollte.

Vanillin und Zucker werden unter Verwendung der Venturi-Düse in den Ansatz eingearbeitet.

Cholindydrogentartrat wird in ca. 3 l heißem Wasser (60°C) gelöst und dem Ansatz zugegeben. Die erneute pH-Kontrolle zeigt, daß der pH nicht unter 6.3 absinkt. Ist dies jedoch der Fall, wird mit Kalilauge oder wässriger Kaliumkarbonatlösung auf einen pH von 6.3-6.5 gepuffert. Danach wird der gesamte Ansatz auf 75°C erwärmt und 10 min gerührt.

Anschließend erfolgt eine 2-stufige Homogenisierung (Stufe 1:150 bar, Stufe 2: 50 bar). Das Konzentrat mit ca. 45% Trockenmasse wird in einen beheizten Vorratstank gepumpt und intensiv gerührt, um Sedimentationen zu vermeiden. Danach erfolgt eine Nacherhitzung mit einem Schabeerhitzer auf 75-80°C und eine 2. Homogenisierung mit 70-100 bar.

Das Konzentrat wird mit einem Sprühturm getrocknet, wobei vorzugsweise mit Düsen getrocknet wird.

| Bedingungen: | Eingangstemperatur | 180 - 190°C |
|---|---|---|
| | Ausgangstemperatur | 90 - 95°C |
| | Düsendruck | 150 bar (120 - 150 bar) |
| | Restfeuchte | max. 2,5°C |
| | Pulvertemperatur | max. 25°C |

Während des Sprühprozesses werden ca. 15-30 % des getrockneten Pulvers erneut in den Sprühbereich des Turmes eingebracht. Dieser Vorgang wird gering gehalten, um eine übermäßige Instantisierung zu vermeiden.

Das auf die oben beschriebene Weise hergestellte Produkt ist gut rieselfähig. Bis zur weiteren Verarbeitung wird es in Polyethylen-/Papiersäcke oder evakuierbaren Behältern abgepackt und aufbewahrt.

1.2 Das so hergestellte Sprühprodukt wird unmittelbar nach der Sprühtrocknung mit der Vitaminmischung gemischt:

Mischrezept:

| | |
|---|---|
| Sprühprodukt | 99,523 % |
| Vitaminmischung | 0,477 % |
| | 100,000 % |

Für 100 kg Mischung werden 99,523 kg Sprühprodukt und 0,4777 kg Vitaminmischung abgewogen und beispielsweise in einem Nauta-Mischer 20 min gemischt.

Danach wird das Mischprodukt bzw. die erhaltene Masse in Polyethylen-/Papiersäcke oder geeignete evakuierbare Container abgenommen und bis zur weiteren Verarbeitung aufbewahrt.

1.3 Die gemäß 1.2 hergestellte Mischung bzw. Masse wird mit einem Monoglycerid mit hohem Schmelzpunkt oder anderen geeigneten hochschmelzenden fettartigen Substanzen (z.B. Boeson® VP, Tegomuls®) als Trennmittel in einem Lödigemischer gemischt.

Mischrezept:

| | |
|---|---|
| Sprühprodukt + Vitaminmischung (= Wirkstoffmischung) | 98 % |
| Trennfett (z.B. Boeson® VP, Tegomuls®) | 2 % |
| | 100 % |

Die Mischzeit im Lödigemischer beträgt 5-10 Minuten. Danach wird die Mischung mit einem Kompaktor (Hutt, Bepex) kompaktiert und mit einem Frewitt-Granulator zu einem Granulat verarbeitet.

Das Granulat wird auf einer Tablettenpresse (z.B. Kilian-Rundläufer, Fette-Rundläufer) zu Tablettenkernen verpreßt mit einem Gewicht von 408-412 mg (entsprechend 400 mg Wirkstoffmischung).

1.4 Dragierung:

Die Tablettenkerne werden in einem Dragierkessel mit fettartigen Substanzen (z.B. Boeson® VP) oberflächenbehandelt und anschließend mit Zucker, Pektin und Gummi arabicum dragiert.

Gewicht pro Dragee:

| | |
|---|---|
| Gummi arabicum | 3,000 mg |
| Zucker | 283,500 mg |
| Pektin | 1,420 mg |
| Boeson® VP (Trennfett) | 0,080 mg |
| Dragee-Decke | 288,000 mg |
| Kerngewicht (Tablettenkern) | 412,000 mg |
| Gesamtgewicht | 700,000 mg |

Die Dragees werden in Dragierkessel hergestellt, die Auftragsmassen mit Warmluft getrocknet.
Die Verpackung erfolgt in Blister.

Die Dragees haben eine sehr gute Haltbarkeit und sind auch in größerer Dosierung pro Tag gut einzunehmen.

Boeson® VP = bemisch aus Mono- und Diglyeriden von gehärteten Fetten

Tegomuls® = Emulgator auf Basis von Glycerin = monostearat

## Analyse:

1 Dragee = 700 mg, enthält 400 mg Wirkstoffmischung

|  | 100 g Dragee | 1 Dragee (=700 mg) | pro 1 g Eiweiß | pro 100 Kcal |
|---|---|---|---|---|
| Eiweiß | 37,6 g | 0,26 g | -- | 11,0 g |
| Eiweißäquivalent *1) | 45,1 g | 0,326 g | -- | 13,0 g |
| (1,2 g Aminosäuren = 1 g Eiweiß) |  |  |  |  |
| Fett | 2,2 g | 0,015 g | -- | 0,6 g |
| Kohlenhydrate | 43,9 g | 0,30 g | -- | 13,0 g |
| Asche | 6,8 g | 0,50 g | -- | 2,0 g |
| Restfeuchte | 2,0 g | -- | -- | -- |
| Energie, kJ | 1469 | 10,1 | -- | -- |
| Kcal | 346 | 2,4 | -- | -- |
| Vitamin A | 0,7 mg | 0,005 mg | 0,019 mg | 0,2 mg |
| Vitamin $B_1$ | 1,0 mg | 0,007 mg | 0,027 mg | 0,3 mg |
| Vitamin $B_2$ | 1,0 mg | 0,007 mg | 0,027 mg | 0,3 mg |
| Vitamin $B_6$ | 2,0 mg | 0,01 mg | 0,05 mg | 0,6 mg |
| Vitamin $B_{12}$ | 2,7 µg | 0,02 µg | 0,07 µg | 0,8 µg |
| Vitamin C | 54 mg | 0,4 mg | 1,4 mg | 15,6 mg |
| Vitamin $D_3$ | 6,4 µg | 0,05 µg | 0,17 µg | 1,9 µg |
| Vitamin E | 6,8 mg | 0,05 mg | 0,18 mg | 2 mg |
| Biotin | 98 µg | 0,7 µg | 2,6 µg | 28,3 µg |
| Ca-D-Pantothenat | 4,8 mg | 0,03 mg | 0,13 mg | 1,4 mg |
| Vitamin $K_1$ | 57 µg | 0,4 µg | 1,5 µg | 16,5 µg |
| Folsäure | 354 µg | 2,5 µg | 9,4 µg | 102 µg |
| Niacinamid | 10 mg | 0,07 mg | 0,27 mg | 2,9 mg |
| Cholin | 120 mg | 0,84 mg | 3,19 mg | 34,7 mg |
| Myo-Inositol | 1,62 mg | 0,011 mg | 0,04 mg | 0,47 mg |

Analyse (Fortsetzung):

| | 100 g Dragee | 1 Dragee (=700 mg) | pro 1 g Eiweiß | pro 100 Kcal |
|---|---|---|---|---|
| Natrium | 730 mg | 5,1 mg | 19 mg | 211 mg |
| Kalium | 1610 mg | 11,3 mg | 43 mg | 465 mg |
| Calcium | 770 mg | 5,4 mg | 20 mg | 223 mg |
| Magnesium | 310 mg | 2,2 mg | 8 mg | 90 mg |
| Phosphor | 460 mg | 3,2 mg | 12 mg | 133 mg |
| Chlorid | 270 mg | 1,9 mg | 7 mg | 78 mg |
| Zink | 13 mg | 0,09 mg | 0,35 mg | 4 mg |
| Kupfer | 2 mg | 0,01 mg | 0,05 mg | 0,06 mg |
| Jod | 150 µg | 1,1 µg | 4 µg | 43 µg |
| Mangan | 2,5 mg | 0,02 mg | 0,07 mg | 0,3 mg |
| Molybdän | 250 µg | 1,3 µg | 7 µg | 75 µg |
| | | | | |
| L-Isoleucin | 2,44 g | 0,02 g | 6,5 g | -- |
| L-Leucin | 4,11 g | 0,03 g | 10,9 g | -- |
| L-Lysin | 3,32 g | 0,02 g | 8,3 g | -- |
| L-Methionin | 0,97 g | 0,007 g | 2,6 g | -- |
| L-Phenylalanin | --- | --- | --- | -- |
| L-Tyrosin | 3,25 g | 0,02 g | 8,6 g | -- |
| L-Threonin | 1,95 g | 0,01 g | 5,2 g | -- |
| L-Tryptophan | 0,76 g | 0,005 g | 2,0 g | -- |
| L-Valin | 2,92 g | 0,02 g | 7,8 g | -- |
| L-Histidin | 0,97 g | 0,007 g | 2,6 g | -- |
| L-Arginin | 1,50 g | 0,05 g | 4,0 g | -- |
| L-Alanin | 1,68 g | 0,01 g | 4,5 g | -- |
| L-Asparaginsäure | 4,13 g | 0,03 g | 11,0 g | -- |
| L-Cystin | 0,97 g | 0,007 g | 2,6 g | -- |
| L-Glutaminsäure | 9,17 g | 0,06 g | 24,4 g | -- |
| L-Glycin | 0,97 g | 0,07 g | 2,6 g | -- |
| L-Prolin | 3,84 g | 0,03 g | 10,2 g | -- |
| L-Serin | 2,16 g | 0,02 g | 5,7 g | -- |

Beispiel 2

Herstellung eines phenylalaninfreinen Diätetikums in Drageeform auf der Basis von ausschließlich essentiellen Aminosäuren.

|  | A Gew.-% | B kg |
|---|---|---|
| Emulgator (Monoglyceride) | 1,000 | 1,000 kg |
| Guar | 0,900 | -- |
| Zucker | 1,850 | 1,854 kg |
| Vanillin | 0,050 | 0,050 kg |
| L-Lysinacetat | 15,000 | 15,075 kg |
| L-Histidin | 4,000 | 4,020 kg |
| L-Isoleucin | 8,800 | 8,844 kg |
| L-Leucin | 15,000 | 15,075 kg |
| L-Methionin | 3,500 | 3,518 kg |
| L-Threonin | 7,000 | 7,035 kg |
| L-Tryptophan | 2,800 | 2,814 kg |
| L-Valin | 11,000 | 11,055 kg |
| L-Tyrosin | 12,000 | 12,060 kg |
| Mineralstoffmischung | 16,600 | 17,600 kg |
| Vitaminmischung | 0,500 | -- |
|  | 100,000 | 100,000 % |
| Tabelle A = Rezeptur | | |
| Tabelle B = Rezeptur für das Sprühprodukt | | |

2.1 Herstellung einer Charge von 100 kg Sprühprodukt:

In einen heizbaren, mit Y-Stral- oder Ultraturrax-Rührer ausgerüsteten Doppelmanteltank werden 100 l kaltes Leitungswasser vorgelegt. Mittels Venturi-Düse wird Guar vollständig in Wasser aufgelöst. Danach werden mittels Venturi-Düse nacheinander zugesetzt:
L-Lysinacetat
L-Isoleucin
L-Leucin
L-Tyrosin
L-Valin
L-Threonin
L-Methionin
L-Histidin
L-Tryptophan
Der Ansatz wird kräftig gerührt und im Kreislauf gepumpt.

Danach wird die Mineralstoffmischung in 50 l heißem Wasser (60°C) vorgelöst und dem Ansatz zugesetzt. Der Ansatz wird auf 75°C angewärmt und 10 Minuten bei dieser Temperatur gerührt.

Der Emulgator wird im Wasserbad bei 70 - 75°C aufgeschmolzen und dem Ansatz zugegeben. Zucker wird zusammen mit Vanillin über die Venturi-Düse dem Ansatz zugesetzt und vollständig aufgelöst.

Der Ansatz wird analog wie im Beispiel 1 beschrieben weiter verarbeitet und sprühgetrocknet.

2.2 Das so hergestellte Sprühprodukt wird unmittelbar nach der Sprühtrocknung mit der Vitaminmischung vermischt:

Mischrezept:

| Sprühprodukt Halbfabrikat | 99,500 % |
|---|---|
| Vitaminmischung | 0,500 % |
|  | 100,000 % |

Für 100 kg Mischung werden 99,500 kg Sprühprodukt und 0,500 kg Vitaminmischung abgewogen und beispielsweise in einem Nauta-Mischer 20 min gemischt. Danach wird das Mischprodukt in geeigneten Containern oder Polyethylen-/Papiersäcken bis zur weiteren Verarbeitung zwischengelagert.

EP 0 554 435 B1

Tablettierung und Dragierung erfolgt dann wie im Beispiel 1 beschrieben.

**Patentansprüche**

1. Verfahren zur Herstellung eines phenylalaninfreien Diätetikums, das neben L-Aminosäuren gegebenenfalls auch Kohlenhydrate, Mineralstoffe, Spurenelemente und/oder Vitamine enthält, für Personen mit Phenylketonurie, insbesondere für juvenile und adulte Personen sowie für schwangere Frauen, in Dragee- oder Tablettenform
wobei man
   a)
      i) die L-Aminosäuren,
      ii) die gegebenenfalls vorhandenen Mineralstoffe und/oder Spurenelemente und
      iii) gegebenenfalls eine Teilmenge oder die Gesamtmenge der Vitamine und/oder der Kohlenhydrate, in Wasser gibt,
   b) den in Stufe a) erhaltenen Naßansatz sprühtrocknet,
   c) das in Stufe b) erhaltene Sprühprodukt gegebenenfalls mit der Restmenge oder der Gesamtmenge der Vitamine und/oder der Kohlenhydrate vermischt,
   wobei man in Stufe a) und/oder in Stufe c) mindestens einen als Emulgator und/oder als Trennmittel dienenden Fettstoff hinzugibt,
   d) die in Stufe c) erhaltene Masse zu Dragee- oder Tablettenkernen verarbeitet und
   e) die Dragee- oder Tablettenkerne mit einem Überzug oder einer Drageedecke versieht.

2. Verfahren nach Anspruch 1,
   wobei man die Gesamtmenge der Vitamine, die insbesondere in einem Kohlenhydrat als Trägermaterial vorgemischt sind, erst in Stufe c) zugibt.

3. Verfahren nach Anspruch 1 oder 2,
   wobei man zur Herstellung des Naßansatzes in Stufe a) kaltes Wasser, insbesondere von 3 bis 15°C, vorlegt, ggf. eine die Viskosität erhöhende Substanz bzw. ein Quellmittel, insbesondere Guar, dazu gibt, anschließend die L-Aminosäuren, die Mineralstoffe und Spurenelemente (sofern vorhanden) sowie die gewünschte Menge an Kohlenhydraten und/oder Vitaminen zugibt, dann den Naßansatz erwärmt, insbesondere auf 70 bis 90° und erst dann den als Trennmittel dienenden Fettstoff, der dem Naßansatz einverleibt werden soll, zu dem erwärmten Naßansatz hinzufügt.

4. Verfahren nach einem der vorhergehenden Ansprüche,
   wobei man bei der Herstellung des Naßansatzes in Stufe a) den pH-Wert nicht unter 6,3 absinken läßt und erforderlichenfalls alkalisch machende Mittel, insbesondere Kaliumhydroxid oder Kaliumcarbonat, zugibt.

5. Verfahren nach einem der vorhergehenden Ansprüche
   wobei man den in Stufe a) erhaltenen Naßansatz vor der Sprühtrocknung homogenisiert und ggf. pasteurisiert sowie nachhomogenisiert.

6. Verfahren nach einem der vorhergehenden Ansprüche,
   wobei man als Fettstoff, der als Emulgator und/oder als Trennmittel dient, mindestens ein Mono-, Di- und/oder Triglycerid einer gesättigten Fettsäure, insbesondere einer Speisefettsäure, mit hohem Schmelzpunkt, insbesondere über 65 °C, oder ein gehärtetes Fett einsetzt.

7. Verfahren nach einem der vorhergehenden Ansprüche,
   wobei man L-Glutaminsäure und L-Asparaginsäure als Alkali- und/oder Erdalkalisalze oder als Salze der Aminosäuren von Arginin und Lysin einsetzt und wobei man insbesondere die nachstehend aufgeführten Aminosäuren in der folgenden Reihenfolge in Stufe a) zum Naßansatz hinzufügt: Natrium-L-Glutamat, L-Arginin-L-Glutamat, L-Lysin-L-Glutamat, K-L-Aspartat, Mg-L-Aspartat, L-Prolin, L-Isoleucin, L-Leucin, L-Tyrosin, L-Threonin, L-Valin, L-Alanin, L-Methionin, L-Cystin, L-Histidin, L-Glycin, L-Tryptophan, L-Serin.

8. Verfahren nach einem der vorhergehenden Ansprüche,
   wobei man den Überzug bzw. die Drageedecke aus Zucker und einem Geliermittel, beispielsweise

12

Gummi Arabicum und Pektin, sowie gegebenenfalls Calciumcarbonat und Trennfett hergestellt.

9. Phenylalaninfreies Diätetikum für Personen mit Phenylketonurie, insbesondere für juvenile und adulte Personen sowie für schwangere Frauen, in Form von Dragees oder Tabletten aus einem Dragee- oder Tablettenkern auf Basis von L-Aminosäuren und gegebenenfalls auch Kohlenhydraten, Mineralstoffen, Spurenelementen und/oder Vitaminen und aus einem Überzug bzw. einer Drageedecke auf dem Dragee- oder Tablettenkern,
dadurch **gekennzeichnet**,
daß der Dragee- oder Tablettenkern mindestens einen lebensmittelrechtlich unbedenklichen Fettstoff enthält, der Überzug bzw. die Drageedecke aus lebensmittelrechtlich unbedenklichen Stoffen zusammengesetzt ist und das Diätetikum nach dem Verfahren gemäß einem der Ansprüche 1 bis 8 erhältlich ist.

10. Phenylalaninfreies Diätetikum nach Anspruch 9,
dadurch **gekennzeichnet**,
daß es lediglich essentielle L-Aminosäuren ( d.h. L-Lysin, L-Isoleucin, L-Leucin, L-Tyrosin, L-Valin, L-Threonin, L-Methionin, L-Histidin und L-Trytophan) mit Ausnahme von Phenylalanin enthält.

11. Masse zur Herstellung eines phenylalaninfreien Diätetikums auf Basis von L-Aminosäuren in Tabletten- bzw. Drageeform, die neben den L-Aminosäuren auch gegebenenfalls Kohlenhydrate, Mineralstoffe, Spurenelementen und/oder Vitamine enthält,
dadurch **gekennzeichnet**,
daß die Masse mindestens einen lebensmittelrechtlich unbedenklichen Fettstoff enthält und
erhältlich ist gemäß dem in Anspruch 1 in den Stufen a), b) und c) beschriebenen Verfahren sowie gegebenenfalls gemäß dem in einem der Ansprüche 2 bis 7 beschriebenen Verfahren.

**Claims**

1. Process for the preparation of phenylalanine-free dietary product, which in addition to L-amino acids optionally also contains carbohydrates, mineral substances, trace elements and/or vitamins, for persons with phenylketonuria, in particular for juvenile and adult persons and also for pregnant women, in pill or tablet form, wherein
   a)
      i) the L-amino acids,
      ii) the optionally present mineral substances and/or trace elements and
      iii) optionally a part amount or the whole amount of the vitamins and/or the carbohydrates, are added to water,
   b) the wet mixture obtained in stage a) is spray-dried,
   c) the spray product obtained in b) is optionally mixed with the residual amount or the whole amount of the vitamins and/or the carbohydrates,
   wherein in stage a) and/or in stage c) at least one fatty substance serving as emulsifier and/or parting agent is added,
   d) the composition obtained in stage c) is processed into the pill or tablet cores and
   e) the pill or tablet cores are provided with a protective layer or a pill covering.

2. Process according to claim 1, wherein the total amount of the vitamins, which in particular are premixed in a carbohydrate as carrier, is first added in stage c).

3. Process according to claim 1 or 2, wherein for the preparation of the wet mixture in stage a) cold water, especially from 3 to 15°C, is taken, a viscosity-increasing agent or a swelling agent, especially guar, is optionally added to this, next the L-amino acids, the mineral substances and trace elements (if present) and also the desired amount of carbohydrates and/or vitamins are added, then the wet mixture is heated, in particular to 70 to 90°C, and only then the fatty substance serving as parting agent, which is to be incorporated into the wet mixture, is added to the heated wet mixture.

4. Process according to one of the foregoing claims, wherein during the preparation of the wet mixture in stage a) the pH value is not allowed to fall below 6.3 and if necessary alkalinising agents, in particular potassium hydroxide or potassium carbonate, are added.

**5.** Process according to one of the foregoing claims, wherein the wet mixture obtained in stage a) is homogenized and optionally pasteurized before the spray-drying and post-homogenised.

**6.** Process according to one of the foregoing claims, wherein as fatty substance which serves as emulsifier and/or as parting agent, at least one mono-, di- and/or triglyceride of a saturated fatty acid, in particular a nutrient fatty acid, with high melting point, in particular above 65°C, or a hardened fat, is used.

**7.** Process according to one of the foregoing claims, wherein L-glutamic acid and L-aspartic acid are used as alkali and/or alkaline earth salts or as salts of the amino acids of arginine and lysine and wherein in particular the amino acids stated below are added to the wet mixture in stage a) in the following order: sodium L-glutamate, L-arginine L-glutamate, L-lysine L-glutamate, K L-aspartate, Mg L-aspartate, L-proline, L-isoleucine, L-leucine, L-tyrosine, L-threonine, L-valine, L-alanine, L-methionine, L-cystine, L-histidine, L-glycine, L-tryptophan, L-serine.

**8.** Process according to one of the foregoing claims, wherein the protective layer or the pill covering is prepared from sugar and a gelling agent, for example gum arabic and pectin, and also optionally calcium carbonate and parting fat.

**9.** Phenylalanine-free dietary product for persons with phenylketonuria, in particular for juvenile and adult persons and also for pregnant women, in pill or tablet form, made from a pill or tablet core based on L-amino acids and optionally also carbohydrates, mineral substances, trace elements and/or vitamins, and from a protective layer or a pill covering on the pill or tablet core, characterised in that the pill or tablet core contains at least one fatty substance acceptable under the foodstuffs law, the protective layer or pill covering is made up of substances acceptable under the foodstuffs law and the dietary product is obtainable by the process according to one of claims 1 to 8.

**10.** Phenylalanine-free dietary product according to claim 9, characterised in that it only contains essential L-amino acids (i.e. L-lysine, L-isoleucine, L-leucine, L-tyrosine, L-valine, L-threonine, L-methionine, L-histidine and L-tryptophan) with the exception of phenylalanine.

**11.** Composition for preparation of an L-amino acid-based phenylalanine-free dietary product in tablet or pill form, which in addition to the L-amino acids optionally also contains carbohydrates, mineral substances, trace elements and/or vitamins, characterised in that the composition contains at least one fatty substance which is acceptable under the foodstuffs law, and is obtainable according to the process described in claim 1 in stages a), b) and c) and also optionally according to the process described in one of the claims 2 to 7.

**Revendications**

**1.** Procédé pour la préparation d'un produit diététique sans phénylalanine qui contient outre les acides L-aminés éventuellement aussi des carbohydrates, des substances minérales, des oligo-éléments et/ou des vitamines, pour des personnes atteintes de phénylcétonurie, plus particulièrement pour des jeunes et des personnes adultes ainsi que les femmes enceintes, sous forme de dragées ou de comprimés, dans lequel :
a) on introduit dans l'eau
    i) les acides L-aminés
    ii) les substances minérales et/ou les oligo-éléments éventuellement présents et
    iii) éventuellement une quantité partielle ou la quantité totale des vitamines et/ou des carbohydrates,
b) on sèche par dispersion la charge humide obtenue à l'étape a),
c) on mélange le produit séché par dispersion obtenu à l'étape b) éventuellement avec une quantité résiduelle ou la totalité des vitamines et/ou des carbohydrates,
en ajoutant à l'étape a) et/ou à l'étape c) au moins un corps gras qui sert d'émulsifiant et/ou d'agent de démoulage,
d) on transforme la masse obtenue à l'étape c) en noyaux de dragées ou de comprimés et
e) on pourvoit les noyaux de dragées ou de comprimés d'un enrobage ou d'une couverture de dragée.

2. Procédé selon la revendication 1 dans lequel on ajoute la quantité totale des vitamines, que l'on a mélangée préalablement, notamment avec un carbohydrate servant de véhicule, seulement à l'étape c).

3. Procédé selon la revendication 1 ou 2 dans lequel pour la préparation de la charge humide à l'étape a), on introduit de l'eau froide, plus particulièrement de 3 à 15°C, en ajoutant éventuellement une substance augmentant la viscosité ou un agent gonflant, notamment le Guar, puis on ajoute des acides L-aminés, des substances minérales et des oligo-éléments (dans la mesure où ils sont présents), ainsi que la quantité désirée de carbohydrates et/ou de vitamines, ensuite on chauffe la charge humide, plus particulièrement à 70 à 90°C et ce n'est qu'après qu'on ajoute à la charge humide chauffée le corps gras qui sert d'agent de démoulage.

4. Procédé selon une des revendications précédentes dans lequel au cours de la préparation de la charge humide à l'étape a) on doit veiller à ce que le pH ne soit pas inférieur à 6,3 et, si nécessaire, on ajoute un agent alcalin notamment l'hydroxyde de potassium ou le carbonate de potassium.

5. Procédé selon une des revendications précédentes dans lequel on homogénéise, avant le séchage par dispersion, la charge humide obtenue à l'étape a) et éventuellement on pasteurise ainsi qu'on effectue une post-homogénéisation.

6. Procédé selon l'une des revendications précédentes dans lequel on utilise comme corps gras qui sert d'émulsifiant et/ou d'agent de démoulage au moins un mono-, di- et/ou triglycéride d'un acide gras saturé, plus particulièrement d'un acide gras alimentaire ayant un point de fusion élevé plus particulièrement supérieur à 65°C ou une graisse durcie.

7. Procédé selon l'une des revendications précédentes dans lequel on utilise l'acide L-glutamique et l'acide L-asparagique sous forme de sel alcalin et/ou alcalino-terreux ou sous forme de sels des acides aminés l'arginine et la lysine et/ou on ajoute notamment les acides aminés indiqué ci-après successivement à l'étape a) à la charge humide : L-glutamate de sodium, L-glutamate de L-arginine, L-glutamate de L-lysine, L-aspartate de K, L-aspartate de Mg, L-proline, L-isoleucine, L-leucine, L-tyrosine, L-thréonine, L-valine, L-alanine, L-méthionine, L-cystine, L-histidine, L-glycine, L-tryptophane, L-sérine.

8. Procédé selon l'une des revendications précédentes où on prépare l'enrobage ou bien la couverture de dragée à partir du sucre et d'un agent gélifiant, par exemple la gomme arabique et la pectine, ainsi qu'éventuellement du carbonate de calcium et un corps gras de démoulage.

9. Produit diététique sans phénylalanine pour personnes atteintes de phénylcétonurie, plus particulièrement pour les adolescents et les personnes adultes ainsi que les femmes enceintes sous forme de dragées ou de comprimés composés d'un noyau de dragée ou de comprimé à base d'acides L-aminés et éventuellement aussi des carbohydrates, des substances minérales, des oligo-éléments et/ou des vitamines et d'un enrobage, respectivement de couverture de dragée sur le noyau de dragée ou de comprimé, caractérisé en ce que le noyau de dragée ou de comprimé contient au moins un corps gras autorisé par la législation des industries alimentaires, l'enrobage ou bien la couverture de dragée est composé de substances inoffensives selon la législation alimentaire et on obtient le produit diététique selon le procédé conforme aux revendications 1 à 8.

10. Produit diététique sans phénylalanine selon la revendication 9, caractérisé en ce qu'il contient uniquement les acides L-aminés essentiels (par exemple L-lysine, L-isoleucine, L-leucine, L-tyrosine, L-valine, L-thréonine, L-méthionine, L-histidine et L-tryptophane) à l'exception de la phénylalanine.

11. Masse pour la préparation d'un produit diététique sans phénylalanine à base d'acides L-aminés sous forme de comprimés ou de dragées qui contient outre les acides L-aminés éventuellement encore des carbohydrates, des substances minérales, des oligo-éléments et/ou des vitamines, caractérisée en ce que la masse contient au moins un corps gras inoffensif d'après la législation des industries alimentaires et que l'on peut obtenir selon le procédé décrit dans la revendication 1 dans les étapes a), b) et c) ainsi que, éventuellement, selon le procédé décrit dans les revendications 2 à 7.